# EUROPEAN PATENT APPLICATION

(11) **EP 3 654 345 A1**
(43) Date of publication of application: **20.05.2020**
(21) Application number: 18205876.8
(22) Date of filing: 13.11.2018
(51) Int. Cl.: G16H 40/60, G08C 17/02, G08C 23/04

(54) **SYSTEM INCLUDING A MEDICAL APPARATUS AND A REMOTE CONTROL DEVICE**

(71) Applicant: TRUMPF Medizin Systeme GmbH + Co. KG, 07318 Saalfeld (DE)
(72) Inventor: KÖHLER, Matthias, Batesville, IN 47006 (US); SCHULZE HORN, Pay, 24105 Kiel (DE); JÄGER, Matthias, Batesville, IN 47006 (US)
(74) Representative: Prüfer & Partner mbB Patentanwälte · Rechtsanwälte

(57) **Abstract**

A system (1) including a medical apparatus (2) and at least one remote control device (3) is provided. The medical apparatus (2) is provided with a first transmitting-receiving device (4) and the at least one remote control device (3) is provided with a second transmitting-receiving device (5). The second transmitting-receiving device (5) is configured to pair with the first transmitting-receiving device (4) and to transmit at different transmitting strengths depending on an operating state of the second transmitting-receiving device (5) with respect to the first transmitting-receiving device (4).

## Description

The invention relates to a system including a medical apparatus and a remote control device and to a method for operating the system.

Usually a pairing process of a medical apparatus and a remote control device is initiated by operating a button of a keypad at the medical apparatus. In the case of an operating table as the medical apparatus, the keypad is usually located at a column of the operating table which is located in a "dirty" area during a surgical intervention. Subsequently, a confirmation command to be sent to the operating table has to be initiated at the remote control device. Hence, pairing of the remote control device and the operating table, e.g. if the batteries of a first remote control are drained during the surgical intervention, is lavishly.

Therefore, document US 9,582,997 B2 discloses a method for pairing the remote control device of the medical apparatus using the remote control device. The operating table comprises a first transmitting-receiving device and the remote control device comprises a second transmitting-receiving device. The second transmitting-receiving device transmits a request command to the first transmitting-receiving device and the first transmitting-receiving device is configured to transmit a device address of the operating table to the second transmitting-receiving device. The second transmitting-receiving device is configured to adopt the received device address and a paired operating state between the first and second transmitting-receiving devices is established.

However, in case of several operating tables in an operating range of the remote control device, it is hard to distinguish with which operating table the remote control device is to be paired.

Therefore, the object underlying the invention is to further develop systems consisting of a remote control device and a medical apparatus of the prior art in order to ensure pairing of the remote control device and a desired medical apparatus and to provide a reliable data transmission between the remote control device and the medical apparatus when paired.

The object is achieved by a system according to claim 1 and method according to claim 10. Advantageous further developments are included in the dependent claims.

According to an aspect of the invention, a system includes a medical apparatus and at least one remote control device, wherein the medical apparatus is provided with a first transmitting-receiving device and the at least one remote control device is provided with a second transmitting-receiving device. The second transmitting-receiving device is configured to pair with the first transmitting-receiving device and to transmit at different transmitting strengths depending on an operating state of the second transmitting-receiving device with respect to the first transmitting-receiving device.

By the different transmitting strengths, the system can ensure a pairing of the remote control device with a desired medical device and a reliable data transmission. The transmitting strength can be adjusted to respective operating conditions of the second transmitting-receiving device with respect to the first transmitting-receiving device, as e.g. unpaired, pairing, and paired.

In an advantageous implementation of the system, the second transmitting-receiving device is configured to transmit at a reduced transmitting strength as long as a pairing of the first transmitting-receiving device and the second transmitting-receiving device is interrupted and to transmit at an increased transmitting strength after the pairing has been established.

By the transmission at the reduced transmitting strength, merely medical apparatuses close to the remote control device are enabled to be paired with the remote control and medical apparatus far away from the remote control device are not paired. By a predetermination of the transmitting strength, a paring range where the medical apparatuses to be paired have to be located can be defined. After completion of the pairing, the second transmitting-receiving device transmits at the increased transmitting strength so that a reliable data transmission between the paired remote control device and medical apparatus is ensured.

In a further advantageous implementation of the system, a ratio of the reduced transmitting strength with respect to a maximum transmitting strength is maximum 5%.

By adjusting the ratio to this value, the paring between the remote control device and the medical apparatus close to the remote control device is ensured.

In a further advantageous implementation of the system, the first transmitting-receiving device is configured to transmit at different transmitting strengths depending on the operating state of the second transmitting-receiving device with respect to the first transmitting-receiving device.

Due to the transmission of the first transmitting-receiving device of the medical apparatus at different transmitting strengths, a more exact determination of the pairing range and an enhanced adjustment of the communication between the medial apparatus and the remote control device is possible.

In a further advantageous implementation of the system, the first transmitting-receiving device is configured to transmit at a reduced transmitting strength as long as the pairing of the first transmitting-receiving device and the second transmitting-receiving device is interrupted and at an increased transmitting strength after the paring has been established.

Due to this characteristic, the pairing range can be further refined since the system can be adjusted to merely accept an actually desired pairing of the medical apparatus and the remote control device and it can ignore a paring request of a remote control device further away and intended to be paired to another device.

In a further advantageous implementation of the system, the first transmitting-receiving device and the second transmitting-receiving device respectively comprise an infrared transmitting-receiving device.

By the provision of the respective infrared transmitting-receiving device, a further improvement of the safety in paring can provided since the two transmitting-receiving devices are to be in an optical connection.

In a further advantageous implementation of the system, at least one of the first transmitting-receiving device and the second transmitting-receiving device comprises a pulse width modulated transmitter, and at least one of the first transmitting-receiving device and the second transmitting-receiving device is configured to vary a pulse width modulation for varying the transmitting strength.

The use of a pulse-width modulated infrared diode enables a simple configuration for transmitting messages at different transmission strengths.

In a further advantageous implementation of the system, the system comprises at least one further remote control device comprising a further second transmitting-receiving device configured to pair with the first transmitting-receiving device.

The option to pair several remote control devices to one medical apparatus enables an option to easily operate the medical apparatus even in case of a failure of the paired remote control device or in case that one of the paired remote control devices cannot be found. The use of a remote control device which has not been paired is quite simple since, merely, a pairing request by, e.g., pushing a button on the remote control device located in the paring range is to be sent to the medical apparatus and after acceptance of the paring request, the pairing is established and the medical apparatus can be operated by the further remote control device.

In a further advantageous implementation of the system, the medical apparatus is configured as an operating table.

In particular in the case of the operating table as the medical apparatus, the easy pairing of the medical apparatus and the intended remote control device and a reliable data transmission is important in order to operate the desired operating table in a reliable manner in, e.g., a case of an emergency.

According to another aspect of the invention, a method for operating the system includes the steps: initiating pairing at the remote control device and transmitting the pairing request by the second transmitting-receiving device at a first reduced transmitting strength, receiving the pairing request and transmitting an identifier of the medical apparatus by the first transmitting-receiving device, receiving the identifier and processing the identifier by the second transmitting-receiving device and pairing the first transmitting-receiving device and the second transmitting-receiving device, and transmitting commands by the second transmitting-receiving device at an increased transmitting strength after having been paired.

By this method, it is enabled that merely medical apparatuses within the pairing range of the remote control device are paired and medical apparatus far away from the remote control device are not paired. After completion of the pairing, the second transmitting-receiving device transmits at the transmitting strength increased with respect to the first reduced transmitting strength so that the reliable data transmission between the remote control device and the medical apparatus is ensured.

In an advantageous implementation of the method, the transmitting of the identifier of the medical apparatus is performed at a second reduced transmitting strength.

Due to this modified step, the pairing range can be further refined since the medical apparatus can be adjusted merely to accept an actually desired pairing with the remote control device and can ignore a paring request of a remote control device further away and intended to be paired to another device.

In a further advantageous implementation of the method, it comprises the step: selecting the medical apparatus to be paired by approaching the remote control device to the medical apparatus up to a predefined distance.

By this proceeding, selecting the medical apparatus to be paired is very simple.

In a further advantageous implementation of the method, the pairing is performed with a further remote control device.

The pairing of the further remote control device and the medical apparatus enables an option to easily operate the medical apparatus even in case of a failure of the paired remote control device or in case that none of the paired remote control devices can be found. The use of a remote control device which has not been paired is quite simple since, merely, a pairing request by, e.g., pushing a button on the remote control device located in the paring range is to be sent to the medical apparatus and, after acceptance of the paring request, the pairing is established and the medical apparatus can be operated by the further remote control device.

Below, the invention is elucidated by an embodiment referring to the attached drawing.

In particular,
- Fig. 1: shows a system of a medical apparatus and a remote control device; and
- Fig. 2: shows a flowchart of a method for operating the system of Fig. 1.

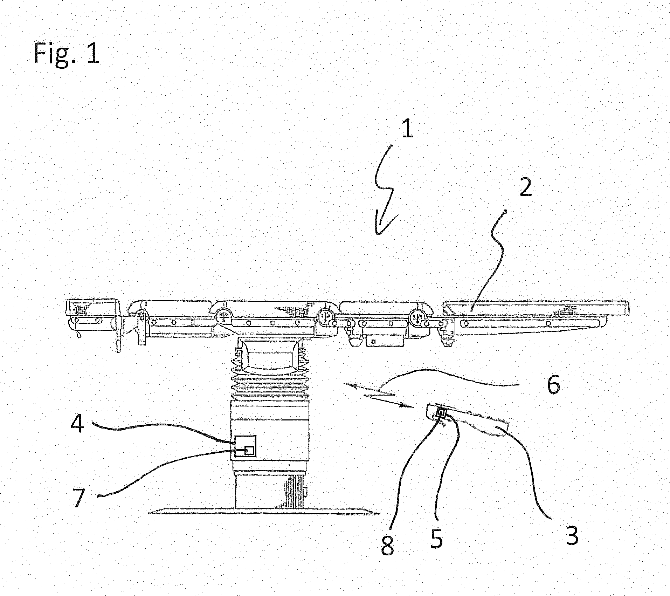

Fig. 1 shows a system 1 of a medical apparatus 2 and a remote control device 3. The medical apparatus 2 is configured as an operating table.

The medical apparatus 2 is provided with a first transmitting-receiving device 4 and the remote control device 3 is provided with a second transmitting-receiving device 5. In an alternative embodiment, at least two remote control devices 3 comprising a further second transmitting-receiving device 5 configured to pair with the first transmitting-receiving device 4 is provided.

The second transmitting-receiving device 5 is configured to pair with the first transmitting-receiving device 4 and to transmit at different transmitting strengths depending on an operating state of the second transmitting-receiving device 5 with respect to the first transmitting-receiving device 4. In particular, the second transmitting-receiving device 5 is configured to transmit at a reduced transmitting strength as long as a pairing 6 of the first transmitting-receiving device 4 and the second transmitting-receiving device 5 is interrupted and at an increased transmitting strength when the pairing 6 is established. By the pairing of the first transmitting-receiving device 4 and the second transmitting-receiving device 5, the medical apparatus 2 and the remote control 3 are paired. A ratio of the reduced transmitting strength with respect to a maximum transmitting strength is 1.7 %. Due to this ratio, a pairing range of about one meter around the remote control device is established. In alternative embodiments, it amounts to maximal 5%.

In this embodiment, the first transmitting-receiving device 4 is configured to transmit at different transmitting strengths depending on an operating state of the first transmitting-receiving device 4 with respect to the second transmitting-receiving device 5. In particular, the first transmitting-receiving device 4 is configured to transmit at a reduced transmitting strength as long as the pairing 6 of the first transmitting-receiving device 4 and the second transmitting-receiving device 5 is interrupted. In contrary, the first transmitting-receiving device transmits at an increased transmitting strength when the pairing 6 is established. In an alternative embodiment, the first transmitting-receiving device 4 does not transmit at different transmitting strengths but at a constant, e.g., maximum, transmitting strength.

The first transmitting-receiving device 4 and the second transmitting-receiving device 5 respectively comprise an infrared transmitting-receiving device 7, 8. The infrared transmitting-receiving devices 7, 8 respectively comprise a pulse width modulated infrared diode as a transmitter, and the first transmitting-receiving device 4 and the second transmitting-receiving device 5 are configured to vary a pulse width modulation for varying the respective transmitting strength. In alternative embodiments, the first and second transmitting-receiving devices 4, 5 are provided with receivers and transmitters based on other physical principles and/or other devices for adjusting the transmission strength and/or merely the second transmitting-receiving device 5 is provided with the device for adjusting the transmission strength.

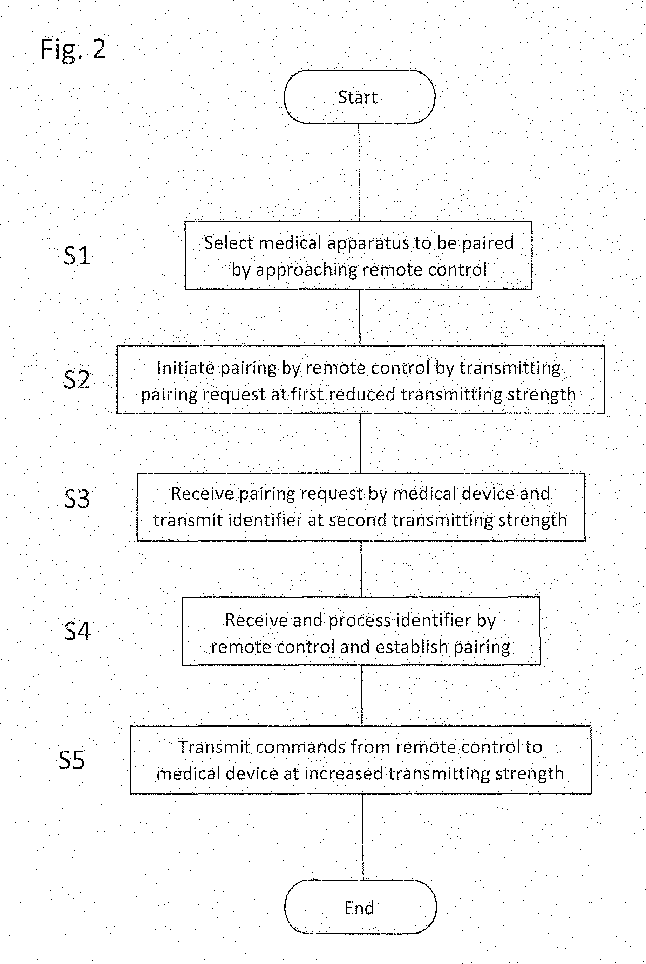

Fig. 2 shows a flowchart of a method for operating the system of Fig. 1.

In use, in step S1, the medical apparatus 2 to be paired is selected by approaching the remote control device 3 to the medical apparatus 2 up to a predefined distance. If the remote control device 3 is alternatively already located within the predefined distance, e.g. attached to a tabletop of the operating table, the approach is not necessary.

In step 2, the second transmitting-receiving device 5 at the remote control device 3 initiates pairing 6 by transmitting a pairing request at a first reduced transmitting strength which is reduced with respect to a maximum transmitting strength. Then, in step S3, the first transmitting-receiving device 4 receives the pairing request and transmits an identifier of the medical apparatus 2 at a second transmitting strength. In step 4, the second transmitting-receiving device 5 receives and processes the identifier and the pairing of the first transmitting-receiving device 4 and the second transmitting-receiving device 5 is established. Then, in step S5, commands are transmitted by the second transmitting-receiving device 5 at an increased transmitting strength.

The transmitting of the identifier of the medical apparatus 2 is performed at a second reduced transmitting strength which is reduced with respect to the maximum transmitting strength. Alternatively, the transmitting of the identifier is performed at the maximum transmitting strength.

As the case may be, the pairing is also performed with a further remote control device 3.

While the invention has been illustrated and described in detail in the drawings and the foregoing description, such illustration and description are to be considered illustrative or exemplary and not restrictive. The invention is not limited to the disclosed embodiments. From reading the present disclosure, other modifications will be apparent to a person skilled in the art. Such modifications may involve other features, which are already known in the art and may be used instead of or in addition to features already described herein. In the claims, the word "comprising" does not exclude other elements or steps, and the indefinite article "a" or "an" does not exclude a plurality.

## Claims

1. A system (1) including a medical apparatus (2) and at least one remote control device (3),
the medical apparatus (2) being provided with a first transmitting-receiving device (4) and the at least one remote control device (3) being provided with a second transmitting-receiving device (5), wherein
the second transmitting-receiving device (5) is configured to pair with the first transmitting-receiving device (4) and to transmit at different transmitting strength depending on an operating state of the second transmitting-receiving device (5) with respect to the first transmitting-receiving device (4).

2. The system (1) of claim 1, wherein
the second transmitting-receiving device (5) is configured to transmit at a reduced transmitting strength as long as a pairing (6) of the first transmitting-receiving device (4) and the second transmitting-receiving device (5) is interrupted and to transmit at an increased transmitting strength after the pairing (6) has been established.

3. The system (1) of claim 2, wherein
a ratio of the reduced transmitting strength with respect to a maximum transmitting strength is maximal 5%.

4. The system (1) of any preceding claim, wherein
the first transmitting-receiving device (4) is configured to transmit at different transmitting strengths depending on the operating state of the second transmitting-receiving device (5) with respect to the first transmitting-receiving device (4).

5. The system (1) of any preceding claim, wherein
the first transmitting-receiving device (4) is configured to transmit at a reduced transmitting strength as long as the pairing (6) of the first transmitting-receiving device (4) and the second transmitting-receiving device (5) is interrupted and at an increased transmitting strength after the pairing (6) has been established.

6. The system (1) of any preceding claim, wherein
the first transmitting-receiving device (4) and the second transmitting-receiving device (5) respectively comprise an infrared transmitting-receiving device (7, 8).

7. The system (1) of any preceding claim, wherein
at least one of the first transmitting-receiving device (4) and the second transmitting-receiving device (5) comprises a pulse width modulated transmitter, and at least one of the first transmitting-receiving device (4) and the second transmitting-receiving device (5) is configured to vary a pulse width modulation for varying the respective transmitting strength.

8. The system (1) of any preceding claim, wherein
the system (1) comprises at least one further remote control device (3) comprising a further second transmitting-receiving device (5) configured to pair with the first transmitting-receiving device (4).

9. The system (1) of any preceding claim, wherein
the medical apparatus (2) is configured as an operating table.

10. Method for operating a system (1) of any preceding claim including the steps:
initiating pairing (6) at the remote control device (3) and transmitting a pairing request by the second transmitting-receiving device (5) at a first reduced transmitting strength;
receiving the pairing request and transmitting an identifier of the medical apparatus by the first transmitting-receiving device (4) after having been paired;
receiving and processing the identifier by the second transmitting-receiving device (5) and pairing the first transmitting-receiving device (4) and the second transmitting-receiving device (5); and
transmitting commands by the second transmitting-receiving device (5) at an increased transmitting strength.

11. Method of claim 10, wherein the transmitting of the identifier of the medical apparatus (2) is performed at a second reduced transmitting strength.

12. Method of claim 10 or 11, including the step:
selecting the medical apparatus (2) to be paired by approaching the remote control device (3) to the medical apparatus (2) up to a predefined distance.

13. Method of anyone of claims 10 to 12, wherein the pairing (6) is performed with a further remote control device (3).
